# EUROPÄISCHE PATENTSCHRIFT

(11) **EP 3 383 319 B1**
(45) Veröffentlichungstag und Bekanntmachung des Hinweises auf die Patenterteilung: **20.03.2019**
(21) Anmeldenummer: 16801791.1
(22) Anmeldetag: 28.11.2016
(51) Int. Cl.: A61F 2/16

(54) **INTRAOKULARLINSE MIT VERSTEIFTER HAPTIK**
INTRAOCULAR LENS HAVING A STIFFENED HAPTIC
LENTILLE INTRAOCULAIRE À HAPTIQUE RAIDI

(30) Priorität: 03.12.2015 DE 102015224140
(43) Veröffentlichungstag der Anmeldung: 10.10.2018
(73) Patentinhaber: Carl Zeiss Meditec AG, 07745 Jena (DE)
(72) Erfinder: PANKIN, Dmitry, 13355 Berlin (DE)
(74) Vertreter: Carl Zeiss AG - Patentabteilung
(86) Internationale Anmeldenummer: PCT/EP2016/079021
(87) Internationale Veröffentlichungsnummer: WO 2017/093194

(56) Entgegenhaltungen:
- US-A- 5 047 051
- US-A- 6 129 760
- US-A1- 2007 093 892
- US-A1- 2011 313 519
- US-A1- 2012 330 415
- US-A1- 2014 330 375

## Beschreibung

Die Erfindung betrifft eine Intraokularlinse mit einem Optikkörper und einer Haptik, die mit dem Optikkörper verbunden ist.

Intraokularlinsen sind üblicherweise mit einem zentralen Optikkörper ausgebildet, der aufgrund seiner Ausgestaltung die optische Abbildungseigenschaft der Intraokularlinse ermöglicht. Radial anschließend an diesen Optikkörper ist üblicherweise eine Haptik ausgebildet, die unterschiedlich gestaltet sein kann. Eine derartige Haptik weist meist mehrere separate Haptikteile auf. Diese können beispielsweise als U-förmige griffartige Elemente mit zwei Enden an den Optikkörper mündend ausgebildet sein. Sie können jedoch beispielsweise auch als freikragende Bügel mit einer C-Form ausgebildet sein.

Darüber hinaus ist es bekannt, dass Intraokularlinsen mit ihrem Optikkörper und der Haptik einstückig aus einem Linsenwerkstoff ausgebildet sind. Der Werkstoff kann dabei ein einheitlicher Werkstoff sein.

Da Intraokularlinsen beim Implantieren in ein Auge gefaltet werden und somit auch in ihrer Größe relativ stark verändert werden, unterliegen sie unterschiedlichsten Anforderungen. Gerade bei kleinschnitttauglichen Linsen ist daher die Anforderung an eine hohe Faltbarkeit sehr hoch. Andererseits dürfen auf das Material der Intraokularlinse nicht jedoch derartige mechanische Krafteinwirkungen beim Implantieren erfolgen, dass sich Risse oder dergleichen bilden.

Um den obigen Anforderungen gerecht zu werden, wird die Haptik relativ filigran ausgebildet, um eine hohe Flexibilität sicherzustelllen. Dies führt jedoch andererseits dazu, dass aufgrund wiederum der materiellen Ausgestaltung der Haptik bei sehr kleiner und dünner Geometrie die Flexibilität unerwünscht zu hoch ist, sodass die Stabilisierungswirkung im implantierten Zustand im Auge eingeschränkt sein kann. Besonders bei einer hydrophoben Intraokularlinse ist es möglich, dass die Haptik so schwach ist, dass sich die Intraokularlinse nicht von selbst in der Mitte des Kapselsackes zentriert, sodass die optische Abbildungswirkung der Intraokularlinse beim Patienten nicht den gewünschten Wert erzielt. Ferner kann es vorkommen, dass aufgrund der unsymmetrischen Lage der Intraokularlinse eine Deformation der Optik erfolgt, sodass auch aus diesem Grund die optische Abbildungswirkung der Intraokularlinse beim Patienten nicht erreicht wird.

Es ist in dem Zusammenhang bekannt, dass sich der Werkstoff der Haptik vom Werkstoff des Optikkörpers dadurch unterscheidet, dass er zum Beispiel härter als der Werkstoff des Optikkörpers ist. Dies hat jedoch bei bekannten Ausführungen zur Folge, dass der Optikkörper und die Haptik aus separaten Komponenten zunächst gefertigt und bereitgestellt werden müssen. Das anschließende Verbinden der Haptik mit dem Optikkörper kann durch Kleben oder Schweißen erfolgen. Dadurch wird zwar eine Intraokularlinse geschaffen, deren Haptik aus einem steiferen Werkstoff als der Optikkörper ist, dies hat jedoch wesentliche Nachteile bei der Fertigung und der Positionsgenauigkeit zwischen der Haptik und dem Optikkörper. Dies kann wiederum dazu führen, dass die Intraokularlinse im Auge nicht ihre vorgesehene Position erreicht, was eine schlechtere Sehqualität für den Patienten bedeutet.

Eine Intraokularlinse, die eine steifere Haptik im Vergleich zu einem Optikkörper aufweist und bei welcher die Haptik an den Optikkörper angeklebt ist, ist beispielsweise aus der EP 1 344 503 A1 bekannt.

Eine Intraokularlinse mit einem Optikkörper und einer Haptik, die mit dem Optikkörper verbunden ist, wobei in der Haptik zumindest ein separater Versteifungsstab angeordnet ist, ist aus der US-A-2007/093892 bekannt.

Darüber hinaus ist aus der US 5 047 051 A eine Intraokularlinse bekannt, bei welcher an den Optikkörper plattenartige haptische Elemente anschließen, die aus einem semisteifen Werkstoff ausgebildet sind. Diese plattenartigen haptischen Teile können dann mit bügelförmigen Teilen zusätzlich ausgestattet sein, die an einem plattenförmigen haptischen Teil befestigt sind. Diese Bügel erstrecken sich auf einem plattenartigen haptischen Teil und sind daran befestigt und erstrecken sich darüber hinaus in radialer Richtung freikragend über den radial äußeren Rand eines plattenartigen haptischen Teils hinweg nach außen. Durch die hohe Flexibilität dieser Bügel soll dann die gesamte Halterung der Intraokularlinse im Auge individuell ermöglicht werden. Die Faltbarkeit einer derartigen Linse ist neben vielfältigen anderen Nachteilen, die insbesondere die Herstellbarkeit betreffen, stark eingeschränkt.

Es ist eine Aufgabe der vorliegenden Erfindung, eine Intraokularlinse zu schaffen, welche bei hoher Flexibilität des Optikkörpers eine verbesserte Stabilität einer relativ filigranen Haptik aufweist.

Diese Aufgabe wird durch eine Intraokularlinse gemäß den Merkmalen des Anspruchs 1 gelöst. Vorteilhafte Weiterbildungen der Erfindung sind Gegenstand der Unteransprüche.

Eine erfindungsgemäße Intraokularlinse weist einen Optikkörper und eine Haptik auf, wobei die Haptik mit dem Optikkörper verbunden ist. In der Haptik ist zumindest ein separater Versteifungsstab angeordnet, der aus einem Werkstoff gebildet ist, welcher eine höhere Steifigkeit als ein Werkstoff der Haptik besitzt. Unter einer Steifigkeit wird ein Widerstand eines Körpers gegen eine elastische Verformung durch eine Kraft oder ein Drehmoment verstanden. Die Steifigkeit kann eine Dehnsteifigkeit, eine Schubsteifigkeit, eine Biegesteifigkeit oder eine Torsionssteifigkeit umfassen.

Durch diese Ausgestaltung der Erfindung ist es somit ermöglicht, dass der Grundkörper der Haptik aus einem relativ flexiblen Basiswerkstoff ausgebildet sein kann. In diesen Grundkörper der Haptik ist dann zumindest ein versteifendes Element angeordnet. Dieses Element ist in seiner Geometrie gemäß der Erfindung spezifiziert und somit als längliches, dünnes Teil gestaltet, indem es stabförmig beziehungsweise strangförmig ausgebildet ist. Durch eine derartige Ausgestaltung lässt sich lokal eine besonders vorteilhafte Armierung der Haptik erreichen. Durch die stabförmige Ausgestaltung ist die Versteifungswirkung individuell gestaltbar und auf spezifische Bereiche der Haptik übertragbar. Durch die Ausgestaltung als Versteifungsstab ist dieses versteifende beziehungsweise armierende Element als solches jedoch relativ klein und filigran ausgestaltet. Die gesamte Größe der Haptik und somit auch der Intraokularlinse wird somit durch eine derartige Ausgestaltung mit einem Versteifungsstab nicht beeinträchtigt, dennoch ist die Steifigkeit örtlich gezielt und gewünscht in der Haptik erhöht, wobei der Basiswerkstoff des Grundkörpers der Haptik hochflexibel sein kann.

Ferner ist gemäß der Erfindung vorgesehen, dass die Haptik einen Aufnahmekanal aufweist, in welchen der Versteifungsstab eingeführt ist. Dies ist dahingehend vorteilhaft, dass somit bereits die örtliche Position und der Verlauf des Versteifungsstabs in der Haptik durch diesen Aufnahmekanal vorgegeben werden kann und somit ein passgenaues Einsetzen des Versteifungsstabs erreicht ist.

Gemäß der Erfindung ist vorgesehen, dass der Aufnahmekanal von einer Oberseite der Haptik ausgehend bis zu einer ersten Tiefe in die Haptik eingebracht ist, wobei der Aufnahmekanal in einem Abschnitt unterbrochen ist, sodass in diesem Abschnitt die Oberseite unverändert vorliegt, wobei in dem Abschnitt von einer der Oberseite der Haptik gegenüber angeordneten Unterseite der Haptik ausgehend eine Ausnehmung in die Haptik bis zu einer zweiten Tiefe eingebracht ist, wobei die Ausnehmung mindestens die Breite des Abschnittes aufweist, wobei die Summe der ersten Tiefe und der zweiten Tiefe mindestens die Summe einer Höhe der Haptik und einer Dicke des Versteifungsstabes ist.

Damit lässt sich der Versteifungsstab auf einfache Weise in den Aufnahmekanal einfädeln sodass er eine stabile und sichere Position in der Haptik einnimmt. Ferner ist es bei der erfindungsgemäßen Intraokularlinse möglich, dass der Versteifungsstab durch das Zusammenwirken mit der an der Unterseite der Haptik eingebrachten Ausnehmung mit dem Aufnahmekanal nur durch Formschluss sicher in der Haptik gehalten ist. Der Einsatz eines Klebstoffes ist nicht zwingend erforderlich. Dies kann bei der Herstellung der Intraokularlinse vorteilhaft sein, da somit keine Zeit für das Aushärten eines eingesetzten Klebstoffes vorgesehen werden muss.

Da die Summe der ersten Tiefe und der zweiten Tiefe mindestens die Summe einer Höhe der Haptik und einer Dicke des Versteifungsstabes beträgt, ist sichergestellt, dass der Versteifungsstab nicht über die Oberseite der Haptik oder Unterseite der Haptik übersteht. Der Versteifungsstab ist somit in der Haptik eingebettet. Damit erfolgt nur ein Gleiten oder Reiben des Haptik-Werkstoffes an der Oberfläche des Kapselsackes eines Patienten. Ein Gleiten oder Reiben des Werkstoffes des Versteifungsstabes mit der Oberfläche des Kapselsackes findet nicht oder nur unwesentlich statt. Bei der Auswahl des Werkstoffes des Versteifungsstabes muss somit keine Rücksicht auf einen möglichst geringen Reibungskoeffizienten genommen werden. Dies ist eine wichtige Voraussetzung dafür, dass sich die Intraokularlinse zuverlässig von selbst in die Mitte des Kapselsackes eines Patienten zentriert.

Ferner bleibt durch diese Anordnung des Versteifungsstabes in dem Aufnahmekanal die Gesamtgröße der Haptik unverändert. Darüber hinaus kann durch diese Ausgestaltung auch verhindert werden, dass sich der Versteifungsstab beim Implantieren der Intraokularlinse in den Kapselsack eines Patienten am Injektor oder im Kapselsack verklemmt oder verspreizt oder auf andere Weise negativ einwirkt.

Mit der erfindungsgemäßen Intraokularlinse ist es möglich, eine Mikroinzision mit einem bisher üblichen Injektor durchzuführen, wobei sich nach der Mikroinzision die Intraokularlinse mit hoher Sicherheit in der für die optische Abbildung wichtigen mittleren Position im Kapselsack hin orientiert. Eine solche zuverlässige Zentrierung der erfindungsgemäßen Intraokularlinse im Kapselsack ist bei einer Intraokularlinse mit einem filigranen freikragenden Haptikbügel (C-Bügel) besonders vorteilhaft.

Es kann auch vorgesehen sein, dass der Versteifungsstab in den Aufnahmekanal eingeklebt ist oder von dem Werkstoff der Haptik umgossen beziehungsweise umspritzt ist. In diesem Fall liegt somit eine stoffschlüssige Verbindung zwischen dem Versteifungsstab und dem Aufnahmekanal beziehungsweise der Haptik vor, so dass eine sehr zuverlässige Position des Versteifungsstabes erreichbar ist.

In einer weiteren Ausgestaltung ist vorgesehen, dass der Aufnahmekanal als Nut ausgebildet ist. Dies bedeutet, dass der Aufnahmekanal zu einer Unterseite der Haptik hin geschlossen ist, was durch einen Nutboden gegeben ist. Zur gegenüberliegenden Oberseite hin ist der Aufnahmekanal dann offen, sodass er diesbezüglich auch zugänglich ist. Dies hat Vorteile bei einem Herstellen der Intraokularlinse, bei welcher der Versteifungsstab in einem separaten Verfahrensschritt eingebracht wird. Eine genaue Lage des Versteifungsstabs in der Haptik ist sehr einfach erreichbar. Bevorzugt weist die Nut einen Hinterschnitt auf, wodurch ein noch sichererer Formschluss zwischen dem Haptikbügel und dem Versteifungsstab erreichbar ist.

Der Versteifungsstab ist bevorzugt aus einem Polymethylmethacrylat (PMMA), einem Polyimid oder einem Polyether Sulfon (PES) gebildet. Ferner ist es möglich, dass als Werkstoff für den Versteifungsstab Polyethylen, Polypropylen, Polyamid oder Polycarbonat oder ein metallischer Werkstoff zum Einsatz kommt, sofern eine Biokompatibilität sichergestellt ist.

Vorteilhafterweise ist vorgesehen, dass die Haptik zumindest mit einem von dem Optikkörper radial abstehenden und freikragenden Haptikbügel mit einem an den Optikkörper mündenden Haptikbügelsockel und einem an den Haptikbügelsockel angrenzenden Haptikbügelhaken ausgebildet ist. Eine derartige geometrische C-förmige Ausgestaltung der Haptik ist im Vergleich zu einer Plattenhaptik relativ biegeschwach. Durch Einsatz des Versteifungsstabes kann die gesamte Haptik eine höhere Steifigkeit auch in dem Fall erreichen, wenn die Haptik aus einem Werkstoff mit einem relativ niedrigen Elastizitätsmodul gebildet ist.

Vorzugsweise ist vorgesehen, dass sich der Versteifungsstab zumindest in dem Haptikbügelhaken erstreckt. Der Haptikbügelhaken liegt bei einer implantierten Intraokularlinse am Rand eines Kapselsackes eines Auges an und positioniert die Intraokularlinse darin. Durch Einsatz des Versteifungsstabes im Haptikbügelhaken kann eine stärkere Anlage der Intraokularlinse im Kapselsack und somit eine zuverlässigere Zentrierung der Intraokularlinse im Kapselsack erreicht werden.

Vorzugsweise ist vorgesehen, dass sich der Versteifungsstab mittig in dem Haptikbügelhaken erstreckt, wodurch in alle Richtungen dieses Haptikbügelhakens eine ausreichende Versteifungswirkung erzielt werden kann.

Insbesondere erstreckt sich der Versteifungsstab zumindest über 80 % der Länge des Haptikbügelhakens, sodass die gleichmäßige und maximale Versteifung über die Länge des Haptikbügelhakens erreicht werden kann.

Vorzugsweise ist vorgesehen, dass ein Versteifungsstab an einem der Optikkörper zugewandten Ende des Haptikbügelsockels angeordnet ist. Durch eine derartige Ausgestaltung kann erreicht werden, dass an dem Übergang zwischen dem Optikkörper und der Haptik eine Versteifung auftritt, sodass eine höhere Biegesteifigkeit der gesamten Haptik erreicht wird.

Es kann vorgesehen sein, dass sowohl ein erster Versteifungsstab in dem Haptikbügelhaken angeordnet ist als auch ein zweiter Versteifungsstab an dem dem Optikkörper zugewandten Ende des Haptikbügelsockels angeordnet ist.

In einer weiteren Ausführung kann vorgesehen sein, dass sich der Versteifungsstab, der sich an einem dem Optikkörper zugewandten Ende des Haptikbügelsockels erstreckt, über eine gesamte azimutale Länge des Haptikbügelsockels um eine optische Hauptachse der Intraokularlinse betrachtet erstreckt. Dies führt zu einer maximalen Versteifung der Haptik.

Mit Angaben "oben", "unten", "vorne", "hinten, "horizontal", "vertikal", "außen", "innen" etc. sind die bei bestimmungsgemäßen Gebrauch und bestimmungsgemäßem Anordnen der Intraokularlinse gegebenen Positionen und Orientierungen angegeben.

Die Erfindung wird nachfolgend anhand schematischer Zeichnungen näher erläutert. Es zeigen:
- Fig. 1: eine Draufsicht einer ersten Ausführungsform einer Intraokularlinse;
- Fig. 2: einen Querschnittsansicht einer Haptik einer Intraokularlinse gemäß der ersten Ausführungsform einer Intraokularlinse;
- Fig. 3: eine Draufsicht einer zweiten Ausführungsform einer Intraokularlinse;
- Fig. 4: eine Draufsicht einer dritten Ausführungsform einer erfindungsgemäßen Intraokularlinse;
- Fig. 5: eine Querschnittsansicht eines Haptikbügelhakens gemäß der dritten Ausführungsform einer erfindungsgemäßen Intraokularlinse;
- Fig. 6: eine Querschnittsansicht eines Haptikbügelhakens gemäß der dritten Ausführungsform der erfindungsgemäßen Intraokularlinse mit einem eingesetzten Versteifungsstab; und
- Fig. 7: eine Draufsicht einer vierten Ausführungsform einer Intraokularlinse.

In den Figuren werden gleiche oder funktionsgleiche Elemente mit den gleichen Bezugszeichen versehen.

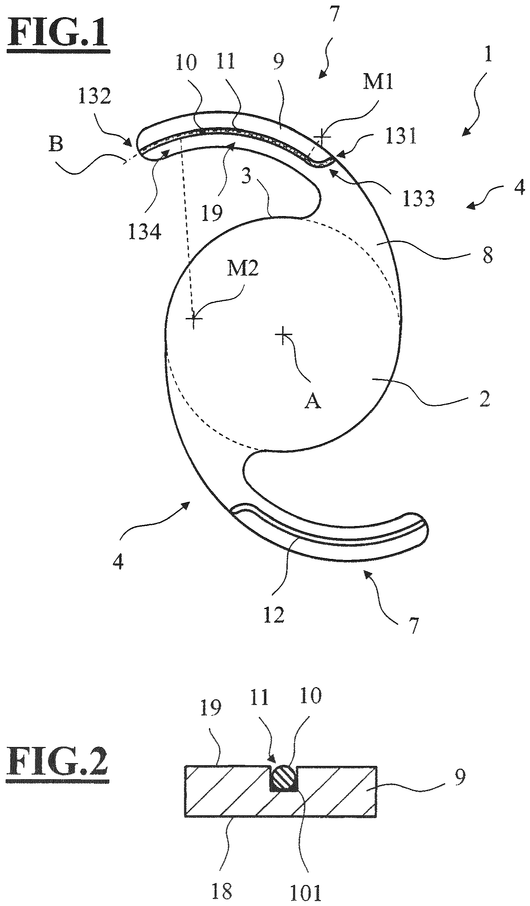

In Fig. 1 ist in einer Draufsicht eine Intraokularlinse 1 gezeigt, die einen Optikkörper 2 aufweist. Die Intraokularlinse 1 und somit auch der Optikkörper 2 weisen eine optische Hauptachse A auf, die senkrecht zu der Ebene verläuft, in der sich der Optikkörper 2 erstreckt. In radialer Richtung zu dieser optischen Hauptachse A grenzt an einen Umfangsrand 3 des Optikkörpers 2 ein Paar von Haptiken 4 an. Dabei ist das Paar von Haptiken 4 so ausgebildet, dass sie sich ausgehend von zwei in der radialen Richtung entgegengesetzt liegenden Positionen entlang des Umfangsrandes 3 des Optikkörpers 2 erstrecken, wobei jede Spitze des Paars von Haptiken 4 ein freies Ende bildet. Eine solche Intraokularlinse 1 wird nach dem Entfernen der Augenlinse in die Linsenkapsel eingesetzt und platziert, um diese zu ersetzen, wobei die Haptiken 4 den Optikkörper 2 in einer vorgegebenen Position innerhalb der Kapsel halten. Das Paar von Haptiken umfasst bei der in Fig. 1 gezeigten Ausführungsform eine obere Haptik und eine untere Haptik, wobei beide Haptiken den gleichen geometrischen Aufbau besitzen.

Die Haptik 4 weist bei dieser Ausführungsform einen von dem Optikkörper 2 radial abstehenden Haptikbügel 7 auf, der einen an dem Optikkörper 2 direkt mündenden Haptikbügelsockel 8 und einen an den Haptikbügelsockel 8 radial nach außen angrenzenden Haptikbügelhaken 9 aufweist. Der Haptikbügelhaken 9 ist somit eine freikragende Komponente der Haptik 4. Die Haptik 4 ist bei dieser Ausführungsform somit C-förmig ausgebildet.

In der in Fig. 1 gezeigten Ausführungsform ist die Intraokularlinse 1 einstückig aus einem gleichen Linsenwerkstoff, insbesondere einem Polymerwerkstoff, ausgebildet.

In der Haptik 4 ist zumindest ein Versteifungsstab 10 angeordnet, der ein zur Haptik 4 separates Teil ist. Der Versteifungsstab 10 ist aus einem Werkstoff gebildet, welcher eine höhere Steifigkeit als der Werkstoff der Haptik 4 und des Optikkörpers 2 aufweist. Der Versteifungsstab 10 ist einstückig ausgebildet und besitzt eine Höhe, die maximal die Höhe der Haptik ist, bevorzugt 50 % der Höhe der Haptik ist, und besonders bevorzugt 20 % der Höhe der Haptik ist. Im Ausführungsbeispiel ist der Versteifungsstab 10 in die Haptik 4, insbesondere in den Haptikbügelhaken 9 eingebettet angeordnet. Dazu umfasst der Haptikbügelhaken 9 einen insbesondere vorgefertigten Aufnahmekanal 11. Der Versteifungsstab 10 ist vollständig innerhalb der Haptik 4 und insbesondere vollständig innerhalb des Aufnahmekanals 11 im Haptikbügelhaken 9 angeordnet, sodass er nicht über die Oberfläche des Haptikbügelhakens 9 übersteht. Wie darüber hinaus in Fig. 1 zu erkennen ist, erstreckt sich der Versteifungsstab 10 über die gesamte azimutale Länge des Haptikbügelhakens 9. In der gezeigten Ausführungsform erstreckt sich der Versteifungsstab 10 nicht in den Haptikbügelsockel 8 hinein.

Es kann vorgesehen sein, dass der Aufnahmekanal 11 radial vollständig geschlossen ist und lediglich an einer in einer Längserstreckung B des Aufnahmekanals 11 betrachteten vorderen Öffnung am proximalen Ende 131 des Haptikbügelhakens 9 und einer hinteren Öffnung am distalen Ende 132 des Haptikbügelhakens 9 offen ist.

Bei der in Fig. 1 gezeigten Ausführung ist der Versteifungsstab 10 von dem proximalen Ende 131 des Haptikbügelhakens 9 beginnend, welches dem Haptikbügelsockel 8 zugewandt ist, entlang des Haptikbügelhakens 9 in Richtung zu dem distalen Ende 132 des Haptikbügelhakens 9 gekrümmt ausgeführt. Die Krümmung am proximalen Ende 131, welche als erste Krümmung 133 bezeichnet ist, besitzt einen ersten Krümmungsmittelpunkt M1, dessen Position vom Versteifungsstab 10 aus betrachtet vom optischen Teil 2 abgewandt ist. An dem distalen Ende 132 des Haptikbügelhakens 9, welches dem Haptikbügelsockel 8 abgewandt ist, weist der Versteifungsstab 10 eine zweite Krümmung 134 auf, jedoch besitzt eine solche zweite Krümmung 134 einen zweiten Krümmungsmittelpunkt M2, dessen Position vom Versteifungsstab 10 aus betrachtet dem optischen Teil 2 zugewandt ist.

In Fig. 2 ist in einer vereinfachten Darstellung ein Querschnitt durch den Haptikbügelhaken 9 einer Intraokularlinse 1 gemäß der Ausführungsform in Figur 1 gezeigt, wobei hier ein zu einer Oberseite 19 hin offener Aufnahmekanal 11 dargestellt ist, der in diesem Beispiel als Nut gestaltet ist. Bei dieser Ausführung ist der Aufnahmekanal 11 insbesondere über seine gesamte Länge als Nut ausgebildet. Es ist zu erkennen, dass der Versteifungsstab 10, der bevorzugt einen kreisförmigen Querschnitt aufweist, vollständig innerhalb des Aufnahmekanals 11 liegt und nicht über die Oberseite 19 übersteht. Er ist bei dieser Ausgestaltung vorzugsweise mit einem Klebstoff 101 in dem Aufnahmekanal 11 befestigt.

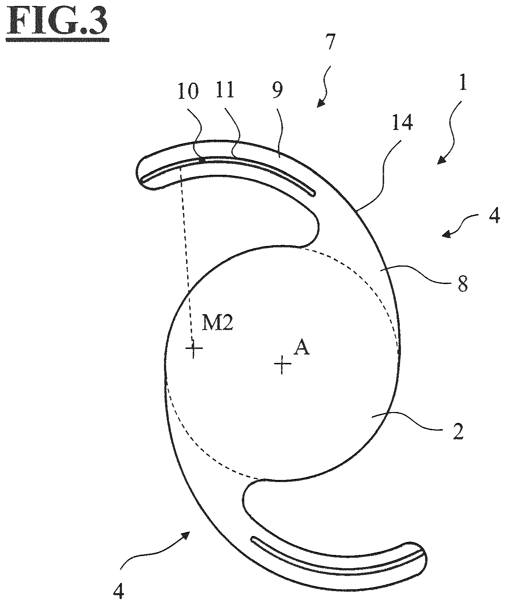

In Fig. 3 ist eine zweite Ausführungsform einer Intraokularlinse 1 gezeigt. Bei dieser Ausgestaltung ist im Unterschied zur Ausführungsform in Fig. 1 die Formgebung und der Verlauf des Versteifungsstabs 10 an dem dem Haptikbügelsockel 8 zugewandten Ende unterschiedlich, und der Versteifungsstab 10 mündet hier nicht an einer Stirnseite 14 der Haptik 4, sondern endet in dem Haptikbügelhaken 9. Der Aufnahmekanal 11 ist somit ein Sackkanal. Der Versteifungsstab 10 besitzt hier eine Krümmung mit einem Krümmungsmittelpunkt M2, dessen Position vom Versteifungsstab 10 aus betrachtet stets dem optischen Teil 2 zugewandt ist.

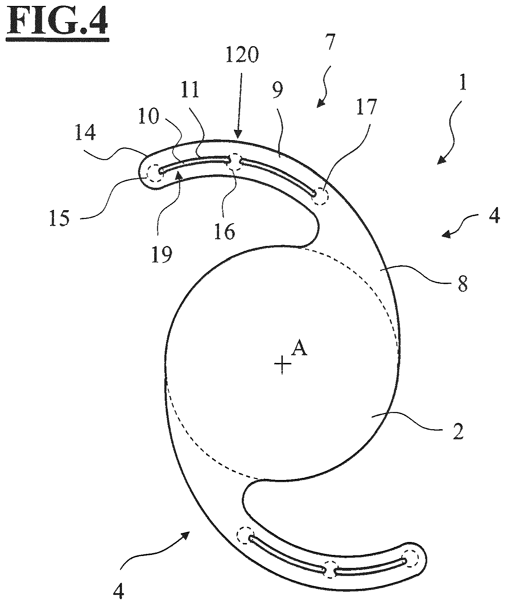

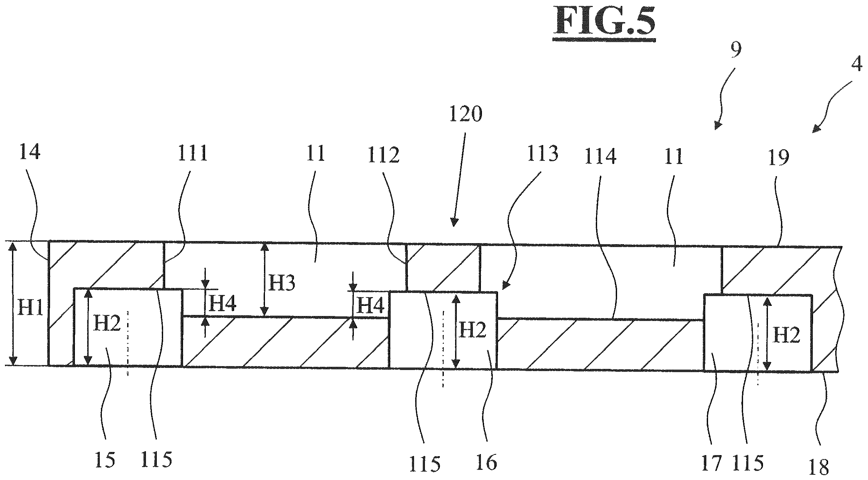

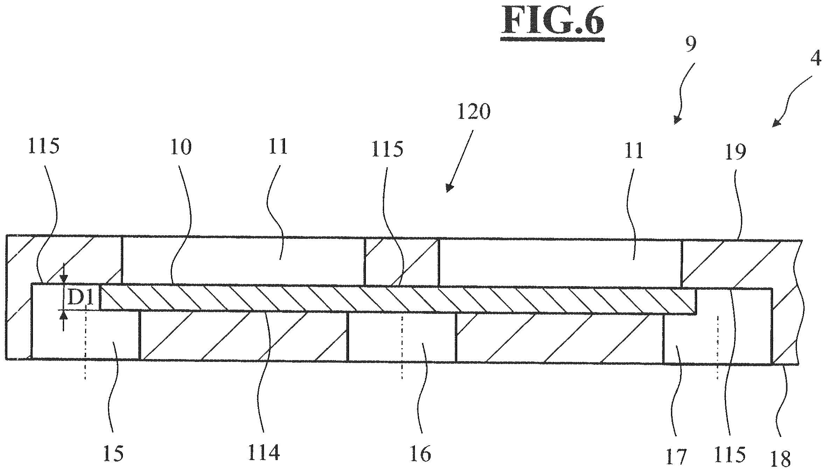

In Fig. 4 ist eine dritte Ausführungsform einer erfindungsgemäßen Intraokularlinse 1 gezeigt. Bei dieser Ausführung ist im Vergleich zu den Darstellungen in Fig. 1 und Fig. 3 der Aufnahmekanal 11 unterschiedlich ausgebildet, vgl. auch Fig. 5 und 6. Der Aufnahmekanal 11 ist an einer Oberseite 19 der Intraokularlinse 1 vorgesehen, jedoch mindestens in einem Bereich 120 unterbrochen. Auf der der Oberseite 19 gegenüberliegenden Unterseite 18 ist eine Ausnehmung 16 vorgesehen, wobei die Ausnehmung 16 gegenüber dem Bereich 120 angeordnet ist. Der Versteifungsstab 10 ist im Aufnahmekanal 11 eingesetzt, wobei er im Bereich 120 in der Ausnehmung 16 verläuft.

Wie in Fig. 4 zu erkennen ist, können an der Unterseite 18 zusätzliche Ausnehmungen 15 und 17 gegenüber den Enden des Aufnahmekanals 11 ausgebildet sein. Die Ausnehmung 16 ist etwa in der Mitte zwischen den beiden Ausnehmungen 15 und 17 angeordnet.

In Fig. 5 ist eine Querschnittsansicht entlang der Längserstreckung B des Aufnahmekanals 11 von einem Haptikbügelhaken 9 der Haptik 4 der dritten Ausführungsform gemäß der Fig. 4 gezeigt. Die Haptik 4 besitzt eine Höhe H1, wobei die Unterseite 18 und die Oberseite 19 der Haptik 4 bevorzugt eben ausgeführt sind und parallel zueinander verlaufen. Ausgehend von der Unterseite 18 sind die Ausnehmungen 15, 16 und 17 in die Haptik 4, bei dieser Ausführungsform in den Haptikbügelhaken 9, eingebracht, und ausgehend von der Oberseite 19 ist in die Haptik 4 der Aufnahmekanal 11 eingebracht.

Die Ausnehmungen 15, 16, 17 besitzen bevorzugt einen kreisförmigen Querschnitt, sodass sie eine Sacklochbohrung darstellen. Der Durchmesser einer Ausnehmung ist so gewählt, dass die Wand der Ausnehmung mindestens an einen linken Rand 111 oder einen rechten Rand 112 des Aufnahmekanals 11 angrenzt. Bevorzugt ist der Durchmesser einer Ausnehmung jedoch so groß, dass die Wand der Ausnehmung 15, 16, 17 in den Aufnahmekanal 11 hineinragt, siehe Bezugszeichen 113 in Fig. 5.

Die Ausnehmungen 15, 16, 17 sind jeweils als Sackloch in die Haptik 4 bis zu einer Tiefe H2 eingebracht, welche kleiner als die Höhe H1 ist. Der Aufnahmekanal 11 ist in der Haptik 4 bis zu einer Tiefe H3 eingebracht, welche kleiner als die Höhe H1 ist. Die Summe der Beträge von H2 und H3 ist größer als der Betrag der Höhe H1. Dies bewirkt, dass die Ausnehmungen 15, 16, 17 teilweise in den jeweiligen Aufnahmekanal 11 eindringen. Bei der in Fig. 5 und 6 gezeigten Ausführungsform ist die Tiefe H2 so gewählt, dass die jeweilige Ausnehmung 15, 16, 17 bis zu einer Tiefe H4 in den jeweiligen Aufnahmekanal 11 eindringt. Die Tiefe H4 ist mindestens gleich einer Dicke D1 des Versteifungsstabes 10, siehe Fig. 6, der an einem Boden 114 des Aufnahmekanals 11 und an einem Boden 115 einer Ausnehmung 15, 16, 17 anliegt. Wenn die Tiefe H4 etwas größer als die Dicke D1 ist, kann der Versteifungsstab 10 ausgehend vom Aufnahmekanal 11 leichter in die Ausnehmungen 15, 16, 17 eingeführt werden. Falls der Versteifungsstab 10 so steif ist, dass ein Einführen nicht möglich ist oder falls ein Einführen nicht erwünscht ist, kann der Versteifungsstab 10 durch ein Spritzgießverfahren mit dem Werkstoff der Haptik 4 umspritzt bzw. umgossen werden, sodass D1 genau gleich H4 ist.

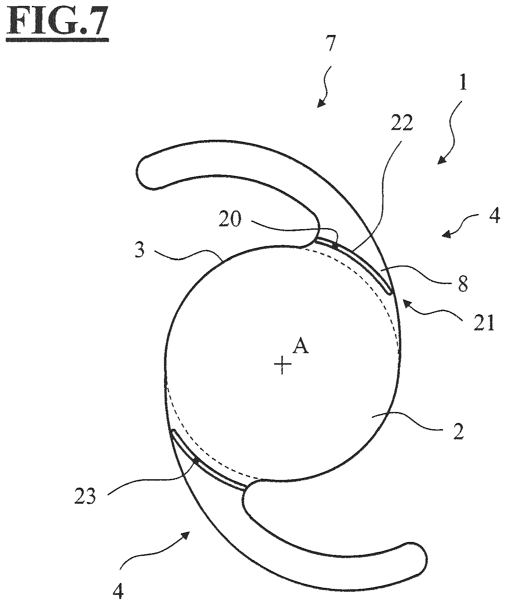

In Fig. 7 ist ein weiteres Ausführungsbeispiel einer Intraokularlinse 1 in einer Draufsicht gezeigt. Bei dieser Ausführung ist vorgesehen, dass nicht in dem Haptikbügelhaken 9, sondern in dem Haptikbügelsockel 8 ein Versteifungsstab 20 eingebettet angeordnet ist. Er ist somit an einem dem Optikkörper 2 zugewandten Ende 21 des Haptikbügelsockels 8 angeordnet und dort in einem Aufnahmekanal 22 eingebettet positioniert. Er erstreckt sich über die gesamte azimutale Länge des Haptikbügelsockels 8 an dieser Stelle und ist darüber hinaus an die Krümmung des Umfangsrandes 3 des Optikkörpers 2 angepasst. An entsprechender Stelle ist ein weiterer Versteifungsstab 23 in der unteren weiteren Haptik 4 eingebettet, welche punktsymmetrisch zur optischen Hauptachse A des Optikkörpers 2 angeordnet ist.

Es kann vorgesehen sein, dass die erste und zweite Ausführungsformen gemäß Fig. 1 bis Fig. 3 und die Ausführungsform gemäß Fig. 7 mit der Ausführungsform gemäß Fig. 4 bis Fig. 6 kombiniert sind. Es ist ebenso möglich, dass in der Haptik 4 zumindest jeweils zwei Versteifungsstäbe 10 und 20 vorgesehen und an diesen spezifischen Stellen angeordnet sind.

## Patentansprüche

1. Intraokularlinse (1) mit einem Optikkörper (2) und einer Haptik (4), die mit dem Optikkörper (2) verbunden ist, wobei in der Haptik (4) zumindest ein separater Versteifungsstab (10, 12; 20, 23) angeordnet ist, der aus einem Werkstoff gebildet ist, welcher eine höhere Steifigkeit als ein Werkstoff der Haptik (4) besitzt, wobei die Haptik (4) einen Aufnahmekanal (11, 22) aufweist, in welchen der Versteifungsstab (10, 12; 20, 23) eingeführt ist,
**dadurch gekennzeichnet, dass**
der Aufnahmekanal (11) von einer Oberseite (19) der Haptik (4) ausgehend bis zu einer ersten Tiefe (H3) in die Haptik (4) eingebracht ist, wobei der Aufnahmekanal (11) in einem Abschnitt (120) unterbrochen ist, sodass in diesem Abschnitt (120) die Oberseite (19) unverändert vorliegt, wobei in dem Abschnitt (120) von einer der Oberseite (19) der Haptik (4) gegenüber angeordneten Unterseite (18) der Haptik (4) ausgehend eine Ausnehmung (16) in die Haptik (4) bis zu einer zweiten Tiefe (H2) eingebracht ist, wobei die Ausnehmung (16) mindestens die Breite des Abschnittes (120) aufweist, wobei die Summe der ersten Tiefe (H3) und der zweiten Tiefe (H2) mindestens die Summe einer Höhe (H1) der Haptik (4) und einer Dicke (D1) des Versteifungsstabes (10, 12) ist.

2. Intraokularlinse (1) nach einem der vorhergehenden Ansprüche,
**dadurch gekennzeichnet, dass**
der Versteifungsstab (10, 12; 20, 23) in den Aufnahmekanal (11, 22) eingeklebt ist oder von dem Werkstoff der Haptik (4) umgossen ist.

3. Intraokularlinse (1) nach einem der vorhergehenden Ansprüche,
**dadurch gekennzeichnet, dass**
der Aufnahmekanal (11, 22) als Nut ausgebildet ist.

4. Intraokularlinse (1) nach einem der vorhergehenden Ansprüche,
**dadurch gekennzeichnet, dass**
die Haptik (4) zumindest einen von dem Optikkörper (2) radial abstehenden und frei kragenden Haptikbügel (7) mit einem an den Optikkörper (2) mündenden Haptikbügelsockel (8) und einem an den Haptikbügelsockel (8) angrenzenden Haptikbügelhaken (9) aufweist.

5. Intraokularlinse (1) nach Anspruch 4,
**dadurch gekennzeichnet, dass**
sich ein Versteifungsstab (10, 12) zumindest in dem Haptikbügelhaken (9) erstreckt.

6. Intraokularlinse (1) nach einem der Ansprüche 4 oder 5,
**dadurch gekennzeichnet, dass**
sich ein Versteifungsstab (10, 12) mittig in dem Haptikbügelhaken (9) erstreckt und/oder sich ein Versteifungsstab (10, 12) zumindest über 80% der Länge des Haptikbügelhakens (9) erstreckt.

7. Intraokularlinse (1) nach einem der Ansprüche 4 bis 6,
**dadurch gekennzeichnet, dass**
ein Versteifungsstab (20, 23) an einem dem Optikkörper (2) zugewandten Ende (21) des Haptikbügelsockels (8) angeordnet ist.

8. Intraokularlinse (1) nach Anspruch 7,
**dadurch gekennzeichnet, dass**
sich der Versteifungsstab (20, 23) über eine gesamte azimutale Länge des Haptikbügelsockels (8) um eine optische Hauptachse (A) der Intraokularlinse (1) erstreckt.

## Claims

1. Intraocular lens (1) having an optics body (2) and a haptic (4), which is connected to the optics body (2), wherein at least one separate stiffening rod (10, 12; 20, 23), which is made from a material having a greater stiffness than a material of the haptic (4), is arranged in the haptic (4), wherein the haptic (4) has a holding channel (11, 22) into which the stiffening rod (10, 12; 20, 23) is inserted, **characterized in that** the holding channel (11) is introduced into the haptic (4) starting from an upper side (19) of the haptic (4) up to a first depth (H3), wherein the holding channel (11) is interrupted in a portion (120), with the result that the upper side (19) is present unchanged in this portion (120), wherein a cutout (16) is introduced into the haptic (4) in the portion (120) starting from a lower side (18) of the haptic (4) that is arranged opposite the upper side (19) of the haptic (4) up to a second depth (H2), wherein the cutout (16) has at least the width of the portion (120), wherein the sum of the first depth (H3) and the second depth (H2) is at least the sum of a height (H1) of the haptic (4) and a thickness (D1) of the stiffening rod (10, 12).

2. Intraocular lens (1) according to one of the preceding claims, **characterized in that** the stiffening rod (10, 12; 20, 23) is adhesively bonded into the holding channel (11, 22) or is encapsulated by the material of the haptic (4).

3. Intraocular lens (1) according to either of the preceding claims, **characterized in that** the holding channel (11, 22) is embodied as a groove.

4. Intraocular lens (1) according to one of the preceding claims, **characterized in that** the haptic (4) has at least one cantilevering haptic arm (7), which radially projects from the optics body (2) and has a haptic arm base (8), which opens at the optics body (2), and a haptic arm hook (9), which adjoins the haptic arm base (8).

5. Intraocular lens (1) according to Claim 4, **characterized in that** a stiffening rod (10, 12) extends at least in the haptic arm hook (9).

6. Intraocular lens (1) according to either of Claims 4 and 5, **characterized in that** a stiffening rod (10, 12) extends centrally in the haptic arm hook (9) and/or a stiffening rod (10, 12) extends at least over 80% of the length of the haptic arm hook (9).

7. Intraocular lens (1) according to one of Claims 4 to 6, **characterized in that** a stiffening rod (20, 23) is arranged at an end (21) of the haptic arm base (8) that faces the optics body (2).

8. Intraocular lens (1) according to Claim 7, **characterized in that** the stiffening rod (20, 23) extends over a total azimuthal length of the haptic arm base (8) about an optical main axis (A) of the intraocular lens (1).

## Revendications

1. Lentille intraoculaire (1) comportant un corps optique (2) et une haptique (4) qui est reliée au corps optique (2), dans laquelle au moins une tige de renforcement séparée (10, 12 ; 20, 23) est disposée dans l'haptique (4) et est formée à partir d'un matériau ayant une rigidité supérieure à celle d'un matériau de l'haptique (4), dans laquelle l'haptique (4) comporte un conduit de réception (11, 22) dans lequel est insérée la tige de renforcement (10, 12 ; 20, 23),
**caractérisée en ce que** le conduit de réception (11) est introduit dans l'haptique (4) depuis un côté supérieur (19) de l'haptique (4) jusqu'à une première profondeur (H3), dans laquelle le conduit de réception (11) est interrompu dans une partie (120) de sorte que, dans cette partie (120), le côté supérieur (19) reste inchangé, dans laquelle, dans la partie (120), un évidement (16) est ménagé dans l'haptique (4) jusqu'à une seconde profondeur (H2) depuis un côté inférieur (18) de l'haptique (4) qui est opposé au côté supérieur (19) de l'haptique (4), dans laquelle l'évidement (16) présente au moins la largeur de la partie (120), dans laquelle la somme de la première profondeur (H3) et de la seconde profondeur (H2) est au moins la somme d'une hauteur (H1) de l'haptique (4) et d'une épaisseur (D1) de la tige de renforcement (10, 12).

2. Lentille intraoculaire (1) selon l'une des revendications précédentes,
**caractérisée en ce que** la tige de renforcement (10, 12 ; 20, 23) est collée dans le conduit de réception (11, 22) ou est encapsulée par le matériau de l'haptique (4).

3. Lentille intraoculaire (1) selon l'une des revendications précédentes,
**caractérisée en ce que** le conduit de réception (11, 22) est réalisé sous la forme d'une rainure.

4. Lentille intraoculaire (1) selon l'une des revendications précédentes,
**caractérisée en ce que** l'haptique (4) comporte au moins un arc haptique (7) s'écartant radialement du corps optique (2) et dépassant librement, avec une base d'arc haptique (8) débouchant sur le corps optique (2) et un crochet en forme d'arc haptique (9) adjacent à la base d'arc haptique (8).

5. Lentille intraoculaire (1) selon la revendication 4,
**caractérisée en ce qu'**une tige de renforcement (10, 12) s'étend au moins dans le crochet en forme d'arc haptique (9) .

6. Lentille intraoculaire (1) selon l'une des revendications 4 ou 5,
**caractérisée en ce qu'**une tige de renforcement (10, 12) s'étend au centre du crochet en forme d'arc haptique (9) et/ou une tige de renforcement (10, 12) s'étend sur au moins 80% de la longueur du crochet en forme d'arc haptique (9).

7. Lentille intraoculaire (1) selon l'une des revendications 4 à 6,
**caractérisée en ce qu'**une tige de renforcement (20, 23) est disposée à une extrémité (21), tournée vers le corps optique (2), de la base de l'arc haptique (8).

8. Lentille intraoculaire (1) selon la revendication 7,
**caractérisée en ce que** la tige de renforcement (20, 23) s'étend autour d'un axe optique principal (A) de la lentille intraoculaire (1) sur toute la longueur azimutale de la base de l'arc haptique (8).
